# EUROPEAN PATENT SPECIFICATION

(11) **EP 4 106 859 B1**
(45) Date of publication and mention of the grant of the patent: **27.11.2024**
(21) Application number: 21709848.2
(22) Date of filing: 11.02.2021
(51) Int. Cl.: A61N 1/05, A61B 5/06, A61B 5/00, A61N 1/362

(54) **A MULTIDIRECTIONAL BALLOON TIPPED CATHETER SYSTEM FOR CONDUCTING HIS BUNDLE SENSING AND PACING**
MULTIDIREKTIONALES BALLONSPITZENKATHETERSYSTEM ZUR LEITUNG DER HIS-BÜNDELERFASSUNG UND -STIMULATION
SYSTÈME DE CATHÉTER À POINTE À BALLONNET MULTIDIRECTIONNEL POUR CONDUIRE LA DÉTECTION ET LA STIMULATION DE FAISCEAU DE HIS

(30) Priority: 18.02.2020 US 202062977973 P
(43) Date of publication of application: 28.12.2022
(73) Proprietor: EAST END MEDICAL, LLC, Lewes, DE 19958 (US)
(72) Inventor: MAINI, Brijeshwar, S., West Palm Beach, FL 33405 (US)
(74) Representative: Patentwerk B.V.
(86) International application number: PCT/US2021/017528
(87) International publication number: WO 2021/167825

(56) References cited:
- WO-A2-2014/036317
- US-A1- 2003 229 386
- US-A1- 2005 065 419
- US-A1- 2009 259 272
- US-A1- 2019 134 412

## Description

### BACKGROUND

Natural Atrioventricular (AV) conducted ventricular contractions utilize the cardiac conduction system. This conduction system is a group of specialized cardiac muscle cells in the walls of the heart that send signals to the heart muscle causing it to contract. The main components of the cardiac conduction system are the sinoatrial (SA) node, AV node, bundle of His, bundle branches, and Purkinje fibers. The SA node (anatomical pacemaker) starts the sequence by causing the atrial muscles to contract. From there, the signal travels to the AV node, through the bundle of His, down the bundle branches (left and right bundles), and through the Purkinje fibers, causing the ventricles to contract. Patients who have AV nodal disease exhibit conduction issues between the atrial and ventricular chambers. Most often, this conduction issue occurs above bundle of His and presents as dyssynchrony of the atrial and ventricular chambers. If the conduction block occurs below the bundle of His and in only one of the left and right bundle branches (LBB and RBB), with intrinsic AV conduction occurring through the remaining intact bundles, but not both, the patient will exhibit a left or right Bundle Branch Block (LBBB or RBBB). Bundle branch blocks are identified by a delayed intrinsic ventricular electrical time. A complete bundle branch block (LBBB or RBBB) is identified as having an electrical activation time greater than 120 ms. Bundle branch blocks are not totally benign, particularly LBBB, and can lead to negative outcomes.

Currently, the standard pacing therapy for patients with AV conduction disease and requiring ventricular pacing, is to place a transvenous lead through the tricuspid valve and into the right ventricle (RV). This RV lead paces the ventricular myocardium which causes a cell by cell slow wave depolarization across the ventricles. This "cell by cell" depolarization of the ventricles utilizing a lead in the right ventricles causes the ventricles to contract in an unnatural way. With conventional RV pacing, the activation sequence of the ventricles is not the same as natural AV conducted contractions, with the right ventricle to depolarizing first and the left ventricle depolarizing slightly after. When patients are right ventricularly (RV) paced at a high percentage, there is an alarming progression of cardiac heart failure and pacing-induced cardiomyopathy. Pacing-induced cardiomyopathy (PICM) is most commonly thought of as a drop in left ventricular ejection fraction (LVEF) in the setting of chronic, high burden right ventricular (RV) pacing. It has been reported that about 20% of patients develop PICM after 3 to 4 years of RV pacing. These negative effects are thought to be a direct result of the unnatural contraction dynamics and resultant ventricular dyssynchrony resulting from RV pacing.

His Bundle Pacing (HBP) has emerged as an alternative to traditional RV pacing. By directly pacing the His bundle, HBP engages electrical activation of both ventricles by means of the cardiac Purkinje fiber network through the natural cardiac conduction system. This type of cardiac pacing may avoid ventricular dyssynchrony and preserve cardiac ejection fraction. Recent studies have also demonstrated the potential of HBP in correcting an underlying left bundle branch block and reversing cardiomyopathy caused by traditional RV pacing. HBP holds promise as an attractive mode to achieve physiological pacing. Widespread adaptation of this technique is dependent on enhancements in technology.

The His bundle lies in most people within the membranous portion of the interventricular septum, with a proportion of the proximal bundle lying on the right atrial portion of the septum, superior to the tricuspid valve annulus. The His bundle is surrounded by fibrous connective tissue rather than myocardium, and then enters the muscular septum and divides to form the right and left bundles. It has been demonstrated that transvenous HBP could reduce QRS duration and normalize electrocardiographic appearances in patients with bundle branch block. There is currently widespread consensus regarding the benefits of HBP and enthusiasm that this therapy may improve patient outcomes.

The current procedural method of achieving HBP involves employing a lead with a fixed screw helix for fixation and one of two catheters to achieve lead position. The fixed helix pacing lead is advanced past the distal end of the catheter while manual catheter manipulation and unipolar mapping utilizing the exposed helix is preformed to locate the His bundle potential signal. Care must be taken when mapping inside the heart with the exposed fixed helix. This helix is fully exposed and can cause local intracardiac surface edema while surface mapping which can mask the His bundle potential. The process of mapping with the exposed helix can often create an acute bundle branch block which may or may not resolve with time. Another pitfall to avoid is the potential for tissue to become stuck in the exposed lead helix which prevents adequate mapping and fixation of the lead helix. The target region for HBP is relatively robust and the current catheters are rather flimsy, which avoids the potential for catheter perforation, although the risk of perforation is possible if the catheter is advanced unintentionally into the incorrect position.

The adoption of HBP has been hindered by the procedural difficulty of achieving good lead position without causing cardiac damage or perforation. The procedural success rates are much lower when compared to traditional RV pacing procedures due to the difficulty in mapping the His potential with the exposed helix and maintaining position while the pacing helix is fixated. The tools currently employed are currently simplistic and lacking the ability to adjust to varying anatomical differences or accurately maintain position within the beating heart during the implant procedure.

US2003/229386 discloses a multidirectional balloon tipped catheter system.

### SUMMARY

The invention is defined by the independent claim 1. Embodiments of the disclosed invention provide a solution through a multidirectional balloon tipped catheter with sensing capability and will lead to increased procedural success and more widespread adoption of His Bundle Pacing (HBP) that has not been used prior and solves this problem.

These advantages and others are achieved, for example, by a multidirectional balloon tipped catheter system for conducting His bundle sensing and pacing. The catheter system includes a multidirectional catheter body having a proximal end and a distal end. The catheter body includes a plurality of curls and flexion points for multidirectional deflections. The catheter body further includes a plurality of lumens which include a pacing lead lumen including an exit port at the distal end and at least one balloon lumen including a balloon port near the distal end. The catheter system further includes an anchor balloon mounted to near the distal end of the catheter body, one or more mapping electrodes mounted to the distal end portion of the catheter body, and a pacing lead placed in the pacing lead lumen. The anchor balloon is in fluid communication with the balloon port and overhangs the distal end of the catheter body by a predetermined distance when the anchor balloon is inflated. The one or more mapping electrodes are configured to sense His bundle potential. The pacing lead is configured to protrude beyond the distal end of the catheter body when the pacing lead is in use.

The anchor balloon may be inflated with a fluid including air, saline, or contrast, and may be configured to be inflated in various sizes. The anchor balloon may be configured to expose the one or more mapping electrodes when the anchor balloon is deflated. The anchor balloon may overhang the distal end of the catheter body by two to three millimeters when the anchor balloon is inflated. The anchor balloon is a hydrophilic balloon.

The one or more mapping electrodes may include a first mapping electrode disposed at the distal end of the catheter body and a second mapping electrode disposed on the catheter body and spaced apart from the first mapping electrode. The first and second mapping electrodes may form a bipolar sensor. A diameter of the pacing lead lumen may be equal to or greater than 0.91 mm. A distance of a distal end of the anchor balloon from the distal end of the catheter body may be in a range of 10 mm to 20 mm when the anchor balloon is deflated. The pacing lead may include a screw helix. The catheter body may be configured to be insertable into a subclavian vein or other vascular access to approach His bundle. The plurality of lumens may further include one or more wiring lumens that house electrical wires connected to the one or more mapping electrodes.

These advantages and others are achieved, for example, by a method for conducting His bundle sensing and pacing with a multidirectional balloon tipped catheter system including a multidirectional catheter body. The method includes inserting the catheter system into a subclavian vein or vascular access, guiding the catheter system towards His bundle, sensing His bundle potential with one or more mapping electrodes disposed near the distal end of the catheter body, positioning a distal end of the catheter body at a location of the His bundle that is determined to be appropriate for pacing, anchoring the distal end of the catheter body at the appropriate location with inflated anchor balloon, and implanting a pacing lead into the appropriate location of the His bundle. The catheter system includes the anchor balloon mounted to the distal end portion of the catheter body, and the anchor balloon is inflated with a fluid supplied through at least one balloon lumen formed in the catheter body. The pacing lead is disposed in a pacing lead lumen formed in the catheter body and advances beyond the distal end of the catheter body while being implanted into the appropriate location of the His bundle.

### BRIEF DESCRIPTION OF THE DRAWINGS

The preferred embodiments described herein and illustrated by the drawings hereinafter be to illustrate and not to limit the invention, where like designations denote like elements.
FIGS. 1A-1C show an embodiment of multidirectional balloon tipped catheter system of the disclosed invention for sensing His bundle and positioning pacing lead.
FIGS. 2A-2C show side views of the distal end portion of the multidirectional balloon tipped catheter system.
FIG. 2D shows a cross-sectional view of the section A-A' of the distal end portion of the multidirectional balloon tipped catheter system shown in FIG. 1C.
FIG. 3 shows an exemplary embodiment of a deflection mechanism that may be employed to control deflections of the distal end portion of the multidirectional balloon tipped catheter system.
FIG. 4, shows a workflow diagram for a method for conducting His bundle sensing and pacing with the multidirectional balloon tipped catheter system.

### DETAILED DESCRIPTION

The following detailed description is merely exemplary in nature and is not intended to limit the described embodiments or the application and uses of the described embodiments. As used herein, the word "exemplary" or "illustrative" means "serving as an example, instance, or illustration." Any implementation described herein as "exemplary or "illustrative" is not necessarily to be construed as preferred or advantageous over other implementations. All of the implementations described below are exemplary implementations provided to enable persons skilled in the art to make or use the embodiments of the disclosure and are not intended to limit the scope of the disclosure, which is defined by the claims. Furthermore, there is no intention to be bound by any expressed or implied theory presented in the preceding technical field, background, brief summary or the following detailed description. It is also to be understood that the specific devices and processes illustrated in the attached drawings, and described in the following specification, are simply exemplary embodiments of the inventive concepts defined in the appended claims. Hence, specific dimensions and other physical characteristics relating to the embodiments disclosed herein are not to be considered as limiting, unless the claims expressly state otherwise.

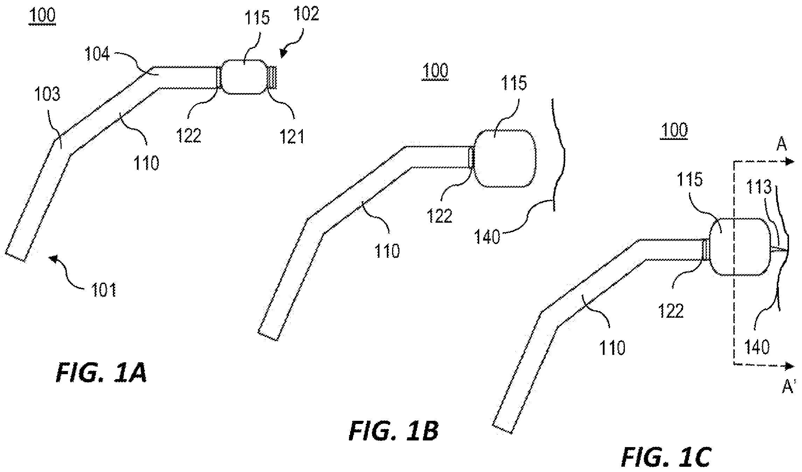

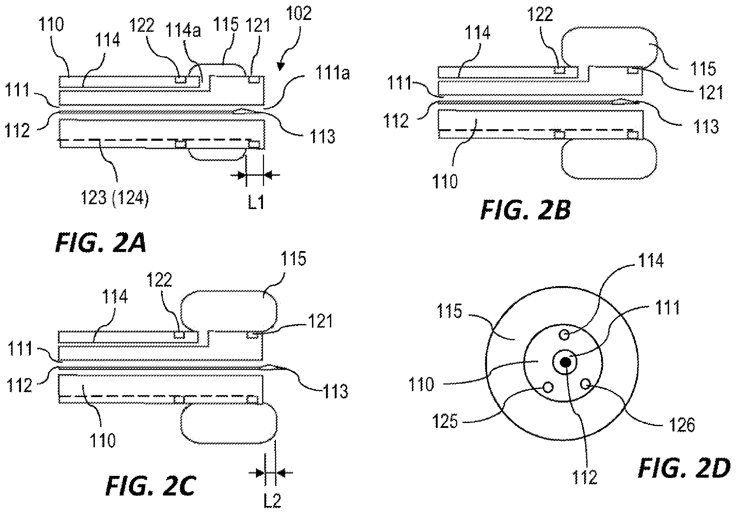

With reference to FIGS. **1A-1C** shown are an embodiment of multidirectional balloon tipped catheter system **100** of the disclosed invention for sensing His bundle and positioning pacing lead. With reference to FIGS. **2A-2C** shown are side views of the distal end portion of the multidirectional balloon tipped catheter system **100.** With reference to FIG. **2D****,** shown is a cross-sectional view of the section A-A' of the distal end portion of the multidirectional balloon tipped catheter system **100.**

The multidirectional balloon tipped catheter system **100** includes a multidirectional or deflectable flexible catheter body **110** that includes a proximal end **101** and a distal end **102.** The catheter body **110** is French sizes, and includes curls and flexion points to be multidirectional or deflectable. For example, the catheter body **110** may include a plurality of flexion points **103, 104** to facilitate the multidirectional deflections or bending. The catheter body **110** has a length sufficient to reach a selected location in a patient's cardiac structures. The catheter body **110** is configured to be insertable into a subclavian vein or other vascular access to approach His bundle. The catheter body **110** includes a plurality of lumens. The plurality of lumens include at least a wire lumen **111** for cord **112** connected to a pacing lead **113.** The wire lumen **111** includes a wire access port (not shown) accessible to an operator at said catheter proximal **101** end and a wire exit port **111a** at said flexible catheter distal end **102,** and a balloon lumen **114** for inflating and deflating at least one anchor balloon **115.** The balloon lumen **114** includes a balloon control port (not shown) for connecting to balloon control device accessible to an operator at the catheter proximal end **101** and a balloon port **114a** near the multidirectional catheter distal end **102.** The plurality of lumens may further include other lumens such as lumens **125, 126** for wires **123, 124** connected to mapping electrodes **121, 122.**

The multidirectional balloon tipped catheter system **100** includes compliant or non-compliant anchor balloon **115** that is mounted on the multidirectional catheter body **110** near the distal end **102** of the catheter body **110.** FIGS. **1A** and **2A** show deflated anchor balloon **115,** and FIGS. **1B** and **2B** show inflated anchor balloon **115** at the distal end **102** portion of catheter body **110.** The anchor balloon **115** is connected to the balloon port **114a** of the catheter body 110, and is in fluid communication through the balloon lumen **114.** Fluid, which is injected or removed at the balloon control port at the proximal end **101,** inflates or deflates the anchor balloon **115** through the balloon lumen **114.**

The anchor balloon **115** may be inflated with air, saline, contrast and other solutions, and may be inflated to various sizes. The anchor balloon **115** is placed at a selected distance from the distal end **102** of the catheter body **110.** For example, when the anchor balloon **115** is deflated, the distance **L1** of a distal end of the anchor balloon **115** from a distal end **102** of the catheter body **110** may be in the range 10 mm (0.4 inches) to 20 mm (0.8 inches). When the anchor balloon **115** is inflated, the anchor balloon **115** may overhang the distal end **102** of the catheter body **110** by a distance **L2** which may be two to three millimeters.

The multidirectional balloon tipped catheter system **100** includes at least one mapping electrode **121** near the distal end **102** of the catheter body **110.** The mapping electrode **121** allows atraumatic mapping of the His bundle potential. The mapping electrode **121** works as a unipolar sensor for detecting and mapping the His bundle potential. In another embodiment, the catheter system **100** may include second mapping electrode **122** that is placed a few millimeters behind the first mapping electrode **121** (toward proximal end **101).** In this configuration, mapping electrodes **121, 122** together work as a bipolar sensor for atraumatic mapping of the His bundle potential, making bipolar sensing possible. The anchor balloon **115** is configured such that the mapping electrode **121** is exposed at the distal end **102** of the catheter body **110** when the anchor balloon **115** is deflated so that mapping the His bundle potential may be performed by using the electrode **121** and/or electrodes **121, 122** to find an appropriate location of the heart tissue **140** for His bundle pacing.

The catheter body **110** may include wiring lumens **125, 126** that house electrical wires **123, 124** connected to the mapping electrodes **121, 122.** The wires **123, 124** at the proximal end **101** of the catheter body **110** may be coupled to an external device that may send signals to or receive signals from the mapping electrodes **121, 122.**

The multidirectional balloon tipped catheter system **100** includes pacing lead **113** that is connected to cord **112** disposed in the lumen **111.** The catheter body **110** includes pacing lead lumen **111** that includes a cord access port (not shown) accessible to an operating device at the proximal end **101** and exit port **111a** at the distal end **102** of the catheter body **110.** The pacing lead lumen **111** may be positioned at a center of the cross-section of the catheter **110** as shown in FIG. **2D****.** The diameter of the pacing lead lumen **111** may be equal to or greater than 0.91 mm (0.035 inches). The pacing lead **113** may be placed inside the pacing lead lumen **111** while mapping His bundle potential and positioning the catheter system **100** against heart tissue **140.** The pacing lead **113** may advance out of the pacing lead lumen **111** beyond the distal end **102** of the catheter body **110** to be placed or implanted on the heart tissue **140.** The pacing lead **113** may have a form of screw helix. FIGS. **1C** and **2C** show the pacing lead **113** advancing out of the distal end **102** of the catheter body **110.**

The anchor balloon **115** may be used in an atraumatic fashion over cardiac structures. The anchor balloon **115** may glide over the cardiac structures, while sensing is performed by using the mapping electrodes **121, 122** to obtain the best site for lead implantation. Once an appropriate location of the heart tissue **140** is determined for His bundle pacing, the catheter system **100** is used as a conduit for implantation of the pacing lead **113.** The anchor balloon **115** may be inflated to anchor the distal end **102** of the catheter body **110** at the appropriate location of the heart tissue **140.** When the distal end **102** of the catheter body **110** with the inflated anchor balloon **115** is positioned and stabilized at the location, the pacing lead **113** may be advanced to be implanted in the heart tissue **140.** Once the pacing lead **113** is in place, the catheter body **110** may be removed using multiple methods which may include slitting and splitting the catheter body, or may be removed by methods that are general practices for lead implantation.

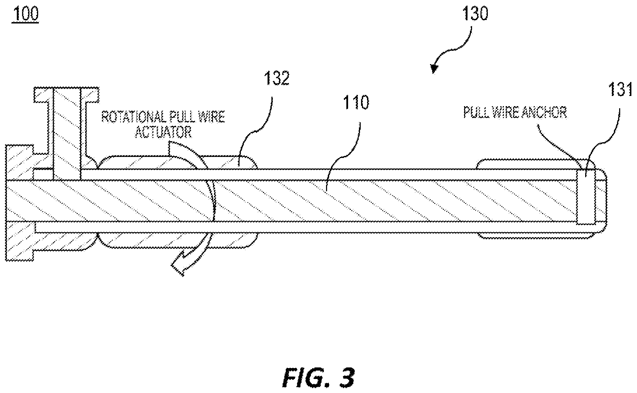

With reference now to FIG. **3****,** shown is an exemplary embodiment of a mechanical deflection device **130** that can be employed at the proximal end **101** portion of the catheter body **110** to control deflections of the distal end portion of the catheter system **100.** Mechanical deflection mechanism may enable distal end of catheter body **110** to be deflected or angulated to various angles with respect to a longitudinal axis (from the proximal end **101** to the distal end **102)** of the catheter system **100.** Mechanical deflection device **130** may include a pull wire anchor **131** affixed to the catheter body **110** and pull wire actuator **132** connected to pull wire anchor **131** with pull wire (not shown). Rotation of pull wire actuator **132,** as shown, may exert force on pull wire anchor **131** that deflects or angulates distal end of the catheter body **110.** Pull wire actuator **132** may be rotated by handle connected thereto (not shown). The deflection device **130** together with the flexion points and curls formed in the catheter body **110** enables the catheter body **110** to easily navigate over heart structures.. U.S. Patent Application Serial No. 17/061,761 filed on October 2, 2020 by the same inventor discloses an improved handle that can be used for the catheter system **100** of the disclosed invention to provide deflections.

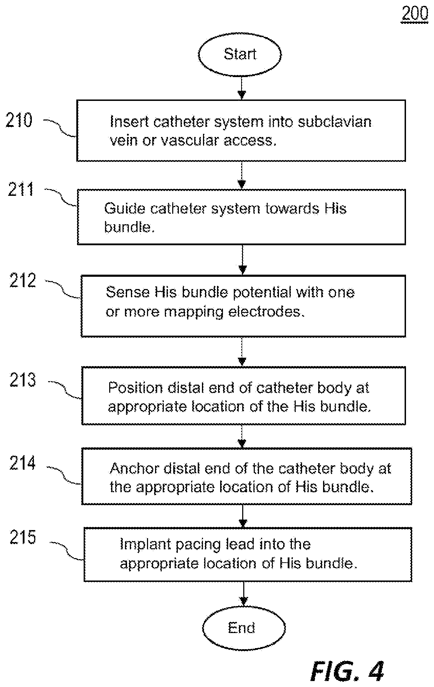

With reference to FIG. **4****,** shown is a workflow diagram for a method **200** for conducting His bundle sensing and pacing with a multidirectional balloon tipped catheter system **100** including a multidirectional catheter body **110.** The catheter system **100** is inserted into a subclavian vein or vascular access, block **210.** The catheter system **100** is guided towards His bundle, block **211.** The catheter system **100** senses His bundle potential with one or more mapping electrodes **121, 122** disposed at a distal end portion of the catheter body, block **212.** The distal end of the catheter body **110** is positioned at a location of the His bundle **140** that is determined to be appropriate for pacing, block **213.** The distal end of the catheter body **110** is anchored at the appropriate location with inflated anchor balloon **115,** block **214.** A pacing lead **113** is implanted into the appropriate location of the His bundle, block **215.** After the pacing lead **113** is implanted, the catheter body **110** with deflated anchor balloon **115** may be removed while leaving the pacing lead **130** in place.

The anchor balloon **115** is atraumatic and allows for use of a more robust catheter designs. The increased rigidity of the catheter body facilitates increased positional precision and an improved procedural success rate. In an embodiment, the anchor balloon **115** may be a hydrophilic balloon with a surface having hydrophilic nature. The catheter system **100** of the disclosed invention provides advantages over the conventional devices. Unlike the conventional devices, the pacing lead **113** of the catheter system **100** of the disclosed invention is not exposed while the catheter system **100** maps the His bundle potential to find an appropriate location of heart tissues for His bundle pacing, preventing any issues that can be caused by exposed screw helix in the conventional devices. The catheter system **100** of the disclosed invention utilizes atraumatic anchor balloon that allows maneuvering of the catheter system **100** over cardiac structures without causing any injuries or damages to heart tissues and also allows to use more rigid multidirectional catheter body, which increases the ability to adjust to varying anatomical differences and to accurately maintain position within the beating heart during the implant procedure.

Since many modifications, variations, and changes in detail can be made to the described preferred embodiments of the invention, it is intended that all matters in the foregoing description and shown in the accompanying drawings be interpreted as illustrative and not in a limiting sense. Consequently, the scope of the invention should be determined by the appended claims.

## Claims

1. A multidirectional balloon tipped catheter system (100) for conducting His bundle sensing and pacing, comprising:
a multidirectional catheter body (110) having a proximal end (101) and a distal end (102) and including a plurality of curls and flexion points (103,104) for multidirectional deflections, wherein the catheter body (110) includes a plurality of lumens (111, 114) comprising:
a pacing lead lumen (111) including an exit port (111a) at the distal end; and
at least one balloon lumen (114) including a balloon port (114a) near the distal end;
an anchor balloon (115) mounted to near the distal end (102) of the catheter body (110), wherein the anchor balloon (115) is in fluid communication with the balloon port (114a) and wherein the anchor balloon overhangs the distal end (102) of the catheter body by a predetermined distance when the anchor balloon is inflated;
one or more mapping electrodes (121,122) mounted to a distal end (102) portion of the catheter body, wherein the one or more mapping electrodes (121,122) are configured to sense His bundle potential,
wherein the anchor balloon (115) is configured to expose the one or more mapping electrodes (121,122) mounted to the distal end (102) portion when the anchor balloon is deflated; and
a pacing lead (113) placed in the pacing lead lumen (111), wherein the pacing lead (113) is configured to protrude beyond the distal end (102) of the catheter body (110) when the pacing lead (113) is in use.

2. The multidirectional balloon tipped catheter system (100) of claim 1 wherein the anchor balloon (115) is inflated with a fluid including air, saline, or contrast.

3. The multidirectional balloon tipped catheter system (100) of claim 1 wherein the anchor balloon (115) is configured to be inflated in various sizes.

4. The multidirectional balloon tipped catheter system of claim 1 wherein the anchor balloon overhangs the distal end of the catheter body by two to three millimeters when the anchor balloon is inflated.

5. The multidirectional balloon tipped catheter system (100) of claim 1 wherein the one or more mapping electrodes (121,122) comprise:
a first mapping electrode (121) disposed at the distal end (102) of the catheter body; and
a second mapping electrode (122) disposed on the catheter body (110) and spaced apart from the first mapping electrode (121), wherein the first and second mapping electrodes form a bipolar sensor.

6. The multidirectional balloon tipped catheter system (100) of claim 1 wherein a diameter of the pacing lead lumen (111) is equal to or greater than 0.91 mm.

7. The multidirectional balloon tipped catheter system (100) of claim 1 wherein a distance (L1) of a distal end of the anchor balloon (115) from the distal end (102) of the catheter body (110) is in a range of 10 mm to 20 mm when the anchor balloon (115) is deflated.

8. The multidirectional balloon tipped catheter system (100) of claim 1 wherein the pacing lead (113) includes a screw helix.

9. The multidirectional balloon tipped catheter system (100) of claim 1 wherein the anchor balloon (115) is a hydrophilic balloon.

10. The multidirectional balloon tipped catheter system (100) of claim 1 wherein the catheter body (110) is configured to be insertable into a subclavian vein or other vascular access to approach His bundle.

11. The multidirectional balloon tipped catheter system (100) of claim 1 wherein the plurality of lumens (111,114) further comprises one or more wiring lumens (125,126) that house electrical wires connected to the one or more mapping electrodes (121,122).

## Patentansprüche

1. Ein multidirektionales Kathetersystem (100) mit Ballonspitze zur Durchführung von Erfassungen und Stimulationen des His-Bündels, umfassend:
einen multidirektionalen Katheterkörper (110), der ein proximalen Ende (101) und ein distales Ende (102) aufweist und mehrere Krümmungen und Biegepunkte (103, 104) für multidirektionale Ablenkungen beinhaltet, wobei der Katheterkörper (110) mehrere Lumen (111, 114) beinhaltet, umfassend:
ein Lumen für eine Schrittmacherleitung (111), das eine Austrittsöffnung (111a) am distalen Ende beinhaltet; und
mindestens ein Ballonlumen (114), das eine Ballonöffnung (114a) in der Nähe des distalen Endes beinhaltet;
einen Ankerballon (115), der in der Nähe des distalen Endes (102) des Katheterkörpers (110) angebracht ist, wobei der Ankerballon (115) in Fluidverbindung mit der Ballonöffnung (114a) steht und wobei der Ankerballon das distale Ende (102) des Katheterkörpers um einen vorbestimmten Abstand überragt, wenn der Ankerballon aufgeblasen ist;
eine oder mehrere Kartierungselektroden (121,122), die an einem Abschnitt des distalen Endes (102) des Katheterkörpers angebracht sind, wobei die eine oder mehreren Kartierungselektroden (121,122) so konfiguriert sind, dass sie das His-Bündel-Potenzial erfassen,
wobei der Ankerballon (115) so konfiguriert ist, dass er die eine oder mehreren am distalen Ende (102) angebrachten Kartierungselektroden (121,122) freilegt, wenn der Ankerballon entleert ist; und
eine Schrittmacherleitung (113), die in dem Lumen für eine Schrittmacherleitung (111) angeordnet ist, wobei die Schrittmacherleitung (113) so konfiguriert ist, dass sie über das distale Ende (102) des Katheterkörpers (110) hervorsteht, wenn die Schrittmacherleitung (113) in Gebrauch ist.

2. Das multidirektionale Kathetersystem (100) mit Ballonspitze nach Anspruch 1, wobei der Ankerballon (115) mit einem Fluid, das Luft, Kochsalzlösung oder Kontrastmittel beinhaltet, aufgeblasen wird.

3. Das multidirektionale Kathetersystem (100) mit Ballonspitze nach Anspruch 1, wobei der Ankerballon (115) so konfiguriert ist, dass er in verschiedenen Größen aufgeblasen werden kann.

4. Das multidirektionale Kathetersystem mit Ballonspitze nach Anspruch 1, wobei der Ankerballon das distale Ende des Katheterkörpers um zwei bis drei Millimeter überragt, wenn der Ankerballon aufgeblasen ist.

5. Das multidirektionale Kathetersystem (100) mit Ballonspitze nach Anspruch 1, wobei die eine oder mehreren Mapping-Elektrode (121,122)umfasst:
eine erste Mapping-Elektrode (121), die am distalen Ende (102) des Katheterkörpers angeordnet ist; und
eine zweite Mapping-Elektrode (122), die auf dem Katheterkörper (110) angeordnet ist und von der ersten Mapping-Elektrode (121) beabstandet ist, wobei die erste und die zweite Mapping-Eelektrode einen bipolaren Sensor bilden.

6. Das multidirektionale Kathetersystem (100) mit Ballonsopitze nach Anspruch 1, wobei ein Durchmesser des Lumens für eine Schrittmacherleitung (111) gleich oder größer als 0,91 mm ist.

7. Das multidirektionale Kathetersystem (100) mit Ballonspitze nachAnspruch 1, wobei ein Abstand (L1) eines distalen Endes des Ankerballons (115) vom distalen Ende (102) des Katheterkörpers (110) im Bereich von 10 mm bis 20 mm liegt, wenn der Ankerballon (115) entleert ist.

8. Das Kathetersystem (100) mit multidirektionaler Ballonspitze nach Anspruch 1, wobei die Schrittmacherleitung (113) eine Schraubenwendel beinhaltet.

9. Das multidirektionale Kathetersystem (100) mit Ballonspitze nach Anspruch 1, wobei der Ankerballon (115) ein hydrophiler Ballon ist.

10. Das multidirektionale Kathetersystem (100) mit Ballonspitze nach Anspruch 1, wobei der Katheterkörper (110) so konfiguriert ist, dass er in eine Vena subclavia oder einen anderen Gefäßzugang eingeführt werden kann, um sich dem His-Bündel zu nähern.

11. Das multidirektionale Kathetersystem (100) mit Ballonspitze nach Anspruch 1, wobei die mehreren Lumen (111,114) ferner ein oder mehrere Verdrahtungslumen (125,126) umfassen, die elektrische Drähte aufnehmen, die mit der einen oder den mehreren Kartierungselektroden (121,122) verbunden sind.

## Revendications

1. Système de cathéter à pointe à ballonnet multidirectionnel (100) pour conduire la détection et la stimulation de faisceau de His, comprenant : un corps de cathéter multidirectionnel (110) ayant une extrémité proximale (101) et
une extrémité distale (102) et comprenant une pluralité de boucles et de points de flexion (103, 104) pour des déflexions multidirectionnelles, dans lequel le corps de cathéter (110) comprend une pluralité de lumières (111, 114) comprenant :
une lumière de sonde de stimulation (111) comprenant un orifice de sortie (111a) à l'extrémité distale ; et
au moins une lumière de ballonnet (114) comprenant un orifice de ballonnet (114a) près de l'extrémité distale ;
un ballonnet d'ancrage (115) monté à proximité de l'extrémité distale (102) du corps de cathéter (110), dans lequel le ballonnet d'ancrage (115) est en communication fluidique avec l'orifice de ballonnet (114a) et dans lequel le ballonnet d'ancrage surplombe l'extrémité distale (102) du corps de cathéter d'une distance prédéterminée lorsque le ballonnet d'ancrage est gonflé ;
une ou plusieurs électrodes de cartographie (121, 122) montées sur une partie d'extrémité distale (102) du corps de cathéter, dans lequel l'une ou plusieurs électrodes de cartographie (121, 122) sont configurées pour détecter un potentiel de faisceau de His,
dans lequel le ballonnet d'ancrage (115) est configuré pour exposer l'une ou plusieurs électrodes de cartographie (121, 122) montées sur la partie d'extrémité distale (102) lorsque le ballonnet d'ancrage est dégonflé ; et
une sonde de stimulation (113) placée dans la lumière de sonde de stimulation (111), dans lequel la sonde de stimulation (113) est configurée pour faire saillie au-delà de l'extrémité distale (102) du corps de cathéter (110) lorsque la sonde de stimulation (113) est utilisée.

2. Système de cathéter à pointe à ballonnet multidirectionnel (100) selon la revendication 1, dans lequel le ballonnet d'ancrage (115) est gonflé avec un fluide comprenant de l'air, une solution saline ou un produit de contraste.

3. Système de cathéter à pointe à ballonnet multidirectionnel (100) selon la revendication 1, dans lequel le ballonnet d'ancrage (115) est configuré pour être gonflé dans plusieurs tailles.

4. Système de cathéter à pointe à ballonnet multidirectionnel selon la revendication 1, dans lequel le ballonnet d'ancrage surplombe l'extrémité distale du corps de cathéter de deux à trois millimètres lorsque le ballonnet d'ancrage est gonflé.

5. Système de cathéter à pointe à ballonnet multidirectionnel (100) selon la revendication 1, dans lequel l'une ou plusieurs électrodes de cartographie (121, 122) comprennent :
une première électrode de cartographie (121) disposée à l'extrémité distale (102) du corps de cathéter ; et
une seconde électrode de cartographie (122) disposée sur le corps de cathéter (110) et espacée de la première électrode de cartographie (121), dans lequel les première et seconde électrodes de cartographie forment un capteur bipolaire.

6. Système de cathéter à pointe à ballonnet multidirectionnel (100) selon la revendication 1, dans lequel un diamètre de la lumière de sonde de stimulation (111) est égal ou supérieur à 0,91 mm.

7. Système de cathéter à pointe à ballonnet multidirectionnel (100) selon la revendication 1, dans lequel une distance (L1) d'une extrémité distale du ballonnet d'ancrage (115) à partir de l'extrémité distale (102) du corps de cathéter (110) est dans une plage de 10 mm à 20 mm lorsque le ballonnet d'ancrage (115) est dégonflé.

8. Système de cathéter à pointe à ballonnet multidirectionnel (100) selon la revendication 1, dans lequel la sonde de stimulation (113) comprend une hélice à vis.

9. Système de cathéter à pointe à ballonnet multidirectionnel (100) selon la revendication 1, dans lequel le ballonnet d'ancrage (115) est un ballonnet hydrophile.

10. Système de cathéter à pointe à ballonnet multidirectionnel (100) selon la revendication 1, dans lequel le corps de cathéter (110) est configuré pour être insérable dans une veine sous-claviculaire ou un autre accès vasculaire pour approcher le faisceau de His.

11. Système de cathéter à pointe à ballonnet multidirectionnel (100) selon la revendication 1, dans lequel la pluralité de lumières (111, 114) comprend en outre une ou plusieurs lumières de câblage (125, 126) qui logent des fils électriques connectés à l'une ou plusieurs électrodes de cartographie (121, 122).
